# EUROPÄISCHE PATENTSCHRIFT

(11) **EP 2 850 244 B1**
(45) Veröffentlichungstag und Bekanntmachung des Hinweises auf die Patenterteilung: **14.09.2016**
(21) Anmeldenummer: 13727532.7
(22) Anmeldetag: 15.05.2013
(51) Int. Cl.: D21C 1/02, B03B 1/00, B09B 3/00, C02F 3/00, C10L 5/40, C13K 1/00, D21C 3/00, D21C 5/00, D21C 7/10

(54) **VERFAHREN ZUR BEHANDLUNG UND SEPARATION VON CELLULOSE-KUNSTSTOFF-MISCHPRODUKTEN**
METHOD FOR TREATING AND SEPARATING MIXED CELLULOSE-PLASTIC PRODUCTS
PROCÉDÉ DE TRAITEMENT ET DE SÉPARATION DE PRODUITS MÉLANGÉS DE CELLULOSE-MATIÈRE PLASTIQUE

(30) Priorität: 15.05.2012 DE 102012104236
(43) Veröffentlichungstag der Anmeldung: 25.03.2015
(73) Patentinhaber: Brandenburgische Technische Universität Cottbus-Senftenberg, 03046 Cottbus (DE); LWG Lausitzer Wasser GmbH & Co. KG, 03046 Cottbus (DE)
(72) Erfinder: BUSCH, Günter, 01156 Dresden (DE); BURKHARDT, Marko, 03046 Cottbus (DE); WEGNER, Jens-Erik, 29478 Höhbeck (DE)
(74) Vertreter: Müller & Schubert
(86) Internationale Anmeldenummer: PCT/EP2013/060005
(87) Internationale Veröffentlichungsnummer: WO 2013/171248

(56) Entgegenhaltungen:
- WO-A1-95/24967
- WO-A1-96/27045
- WO-A1-97/37008
- WO-A2-03/043939
- US-A- 4 990 244

## Beschreibung

Die vorliegende Erfindung betrifft ein Verfahren zur Behandlung und Separation von Cellulose-Kunststoff-Mischprodukten, mit dem Verfahren hergestellte Erzeugnisse sowie die Verwendung derselben.

Ein aktuelles Problem unserer Zeit ist die Überalterung der Gesellschaft, sowohl in Deutschland als auch in anderen europäischen Staaten. Diese Entwicklung führt zu einer ansteigenden Anzahl von Pflegefällen und damit auch der Unterbringung in Pflege- und Altenheimen. Waren es im Jahre 2008 noch 760.000 vollstationäre Pflegeplätze, so werden für das Jahr 2025 bereits 1.160.000 Plätze prognostiziert [1]. Bessere medizinische Versorgung und eine generelle höhere Lebenserwartung führt zu dieser Entwicklung. Während im Jahr 2008 etwa 20 % der Bevölkerung älter als 65 Jahre war, werden es im Jahr 2060 bereits 34 % der Bevölkerung sein [2]. Aus dem Blickwinkel der Abfallwirtschaft führt diese Entwicklung zu einem Anstieg des Aufkommens von sogenanntem Inkontinenzmaterial (IKM), das hauptsächlich aus Windeln, aber auch aus Bandagen, Einwegtüchern und verschiedenen Fehlwürfen zusammengesetzt ist. IKM wird auch als absorbierende Hygieneartikel bezeichnet.

Das IKM wird in Säcken getrennt gesammelt. Die Entsorgung erfolgt im Allgemeinen gemeinsam mit dem Restmüll. Nach der thermischen bzw. mechanisch-biologischen Behandlung erfolgt die Deponierung der Reststoffe. Eine durch ein Cottbuser Pflegeheim (109 Betten) durchgeführte Studie ergab eine IKM Produktion von 0,46 t/a [3].

Es wird erwartet, dass das IKM-Aufkommen in Pflegeheimen in den nächsten Jahren um über 50 % ansteigen wird. Zusätzlich entsteht vergleichbares Material in Krankenhäusern oder in Haushalten mit Kindern von unter 3 Jahren. Diese Entwicklung führt sowohl zu steigenden Behandlungs- und Entsorgungskosten als auch zum Verlust von Energie und stofflicher Ressourcen durch die Anwendung bisheriger Entsorgungswege.

Im Stand der Technik sind eine Reihe von Verfahren und Vorrichtungen bekannt, welche zur Abfallaufbereitung oder -behandlung sowie zur Behandlung und Gewinnung von Cellulose und zum Recycling von Kunststoffen dienen.

Die US 5,777,086 A betrifft nun ein Verfahren zur Extraktion von Lignin aus einer Pflanzenfasern enthaltenden wässrigen Mischung, wobei das Verfahren alkalisch geführt wird.

Die WO 2003/0043939 A2 betrifft ein Verfahren zur Behandlung bioabbaubarer organischer Anfälle. Auch hierbei wird das Verfahren alkalisch geführt. Derartige Abfälle bestehen aus organischen Haus- und Gartenabfällen, Lebensmittelresten und Feststoffen aus der Abwasserbehandlung.

Die US 7,985,778 B2 beschreibt ein Verfahren zur Rückgewinnung von Monomeren aus synthetischen Polymeren, insbesondere aus Polyestern wie PLA, PCL, PBS, PHB und PET.

WO 2012/045298 A2 betrifft ein Verfahren zur Aufbereitung von holzhaltigem Grünschnitt zur Papierherstellung in einem Pulper.

GB 2475951 A beschreibt ein Abfallbehandlungsverfahren für Haus- und Gewerbemüll, dessen Ziel die thermische Verwertung und/oder Pyrolyse des Abfalls ist.

In der DE 69727437 T2 werden eine Vorrichtung und ein Verfahren zur Nitratentfernung aus wässrigen Medien beschrieben, wobei man permeable Polymerkügelchen mit Bakterien und einer Kohlenstoffquelle wie Cellulose, beladen und dann in das wässrige Medium einbringt.

WO 9627045 betrifft ein Verfahren zur Separation von cellulose-kruststoff-Mischiprodukten wo Waschmaschinen eingesetzt werden.

Der gegenwärtige Weg der thermisch/energetischen Abfallnutzung stellt eine endgültige Materialvernichtung dar und ist in der Energiebilanz eindeutig schlechter. Ein werkstoffliches Recycling oder rohstoffliche Verfahren sollten vorgezogen werden [4].

Die Einwegwindeln, die den Hauptanteil des IKM darstellen, bestehen zu 18 % aus Kunststoff und zu 60 % aus Papier. Diese beiden Materialien bieten sich für eine Wiederverwertung an. Der Kunststoff ist eine Mischung aus einem Polypropylenvlies (11 %) und der äußeren Polyethylenfolie (7 %). Der innere Kern besteht aus Cellulose und dem Superabsorbierenden Polymer (SAP). Nach dem Gebrauch der Windeln enthalten sie zudem die Exkremente und Urin. Gegebenenfalls können Spuren von Arzneimittelrückständen wie Antibiotika, Hormone, Röntgenkontrastmittel oder Desinfektionsmittel enthalten sein, deren Verbreitung in die Umwelt und den Stoffkreislauf vermieden werden muss. Ein integrierter Prozess ist erforderlich, der die Behandlung des Inkontinenzmaterials erlaubt und zugleich eine stoffliche und energetische Verwertung ermöglicht.

Mit dem steigenden Energiebedarf in den vergangenen Jahren, bei gleichzeitiger Steigerung der Rohstoffbeschaffungskosten hat die Vergärung von Biomasse und insbesondere organischer Reststoffe zur Produktion von Biogas zugenommen. Vielfältige Einsatzstoffe bieten sich als Substrat in der Fermentation an, wobei jedoch in Bezug auf ihre Eignung eine starke Abhängigkeit von ihrer Zusammensetzung vorliegt. Biomasse besteht im Allgemeinen aus den unlöslichen Verbindungen wie Cellulose Lignocellulose, Hemicellulose und den löslichen Verbindungen wie Eiweißen, Fetten, Mineralstoffen und löslichen Kohlenhydraten (Zucker, Stärke, Pektin, Inulin). Die Zusammensetzung des Substrates bestimmt aufgrund der unterschiedlichen Abbaubarkeit die Umsatzgeschwindigkeit und damit die Biogasproduktivität.

Der anaerobe Abbau erfolgt als vierstufiger Prozess. Diese sind die Hydrolyse, Acidogenese, Acetogenese und Methanogenese. An diesen Schritten sind unterschiedliche Mikroorganismen und verschiedenen Enzyme beteiligt. Im ersten Schritt, der Hydrolyse, erfolgt die Überführung der unlöslichen Verbindungen in eine lösliche Form. Die Hydrolyse ist sehr bedeutsam, da der weitergehende biologische Stoffumsatz lediglich in einer wässrigen Phase erfolgen kann. Je nach Zusammensetzung der Biomasse ist die Hydrolyse der geschwindigkeitsbestimmende Schritt der gesamten Abbaukette [5]. Substrate mit hohen Anteilen an Cellulose und lignocellulosehaltige Verbindungen benötigen daher eine lange Verweilzeit in den Fermentern, wobei der Methanertrag gering ist. Erst aus den gelösten Zwischenprodukten Zucker, organische Säuren, Kohlenstoffdioxid und Wasserstoff erfolgt in den weiteren Schritten die Umsetzung durch methanogene Archaeen zu Methan und Kohlenstoffdioxid als Hauptbestandteile des Biogases.

In Hinblick auf den besseren Zellaufschluss und die Beschleunigung der Hydrolyse, erfolgten bereits eine Vielzahl von Untersuchungen, beispielsweise durch sauren oder alkalischen Aufschluss, mechanische Partikelgrößenverringerung, Wärme- oder Kältebehandlung. Dabei hat sich insbesondere für den Aufschluss von Cellulose und Lignocellulose die Thermodruckhydrolyse "TDH" bei Temperaturen ab 150 °C und Drücken von 1,2-3,0 MPa als vielversprechend erwiesen [6]. Schwer abbaubare Biopolymere werden dabei zu kurzkettigen Monomeren reduziert. Cellulose ist ein natürliches Polymer (Polysaccharid), das durch eine β-1,4-glycosidische Bindung des Monosaccharids Cellobiose (Disaccharid) zusammengesetzt ist. Cellobiose besteht aus dem Einfachzucker Glukose. Mit Hilfe von Temperatur- und/oder Säureeinwirkung kann Cellulose zu Glukose durch Spaltung der glycosidischen Bindung abgebaut werden. Als Produkte der TDH liegen daher vorwiegend Glukose, Di- und Polysaccharide vor.

Mit dem IKM liegt jedoch keine reine Cellulose vor. Sowohl die enthaltenen Kunststoffe, aber auch die Beladung, das SAP und die Medikamente haben einen Einfluss auf die optimale Prozessführung der TDH. Zudem kann eine möglicherweise negative Wirkung auf den nachfolgenden biologischen Prozess nicht ausgeschlossen werden.

Bislang ist kein Verfahren bekannt, in dem die Kunststoffabtrennung, der Celluloseaufschluss, die Hygienisierung und Pharmakainhibierung in einem Schritt erfolgt. Bislang sind mehrere Behandlungsschritte einschließlich der Anlagen erforderlich, die diese jeweilige Zielstellung erbringt. Damit gehen hohe Investitions- und Betriebskosten einher.

Auch erfolgt zum Teil keine Separation oder Abtrennung. Der Kunststoff wird beispielsweise mit der Organik durch die anaerobe Behandlung (Vergärung) hindurchgeschleust. Es kommt zur Schwimmdeckenbildung, einem höheren notwendigen Fermentervolumen, einem höheren Energieverbrauch beim Aufheizen des größeren Volumens und ggf. zu Materialtransport- und Mischproblemen.

Die Hygienisierung und Pharmakainhibierung erfolgt vor dem Einsatz in einer Biogasanlage nicht. In der Anlage besteht dann die Gefahr der Hemmung, bzw. des Austrages von Krankheitserregern und umweltrelevanten Arzneimitteln. Die Hygienisierung und Pharmakainhibierung wird zum Teil durch thermische Behandlung gelöst, wobei aufgrund des geringen Heizwertes eine Stützfeuerung erforderlich ist. Eine Verwertung der Cellulose in anaeroben (Biogasbildung) oder aeroben Prozessen (Denitrifikation) ist nicht möglich. Eine stoffliche Nutzung ist ebenfalls unmöglich.

Aufgabe der vorliegenden Erfindung ist es daher, die Nachteile des Standes der Technik zu überwinden und ein Verfahren zur Trennung bzw. Separation der Cellulose-Kunststoff-Mischprodukte zur Verfügung zu stellen, welches die nachfolgende stoffliche und/oder energetische Nutzung der separierten Produkte ermöglicht.

Die Aufgabe wird durch ein Verfahren mit den Merkmalen des Hauptanspruchs gelöst. Vorteilhafte Ausgestaltungen des erfindungsgemäßen Verfahrens sind in den abhängigen Unteransprüchen gekennzeichnet.

Gegenstand der Erfindung ist somit ein Verfahren zur Separation von Cellulose-Kunststoff-Mischprodukten, wobei man die folgenden Schritte durchführt:
a) Anmaischen der Cellulose-Kunststoff-Mischprodukte mit Wasser;
b) Einbringen der im Schritt a) erzeugten Maische in einen Rührautoklaven und Verschließen des Rührautoklaven;
c) Erwärmen der Maische unter fortgesetztem Rühren mit einer Drehzahl von 50 bis 350 min⁻¹, auf eine Temperatur von 150 bis 250 °C, bei einem Druck von 1,2 bis 3,0 MPa;
d) Halten der Temperatur und des Drucks der im Schritt c) erwärmten Maische unter fortgesetztem Rühren für eine Haltezeit von 0,3 bis 6 Stunden, wobei die Cellulose hydrolysiert und die Kunststoffe zu kugelförmigen Gebilden agglomerieren;
e) Abkühlen, Entspannen und Öffnen des Rührautoklaven.

Vorteilhaft ist ein erfindungsgemäßes Verfahren, bei dem im Schritt c) die Rührerdrehzahl zwischen 100 bis 300 min⁻¹, bevorzugt zwischen 120 bis 290 min⁻¹ und besonders bevorzugt zwischen 140 bis 260 min⁻¹ liegt.

Weiterhin vorteilhaft ist ein erfindungsgemäßes Verfahren, bei dem im Schritt c) die Temperatur zwischen 180 bis 220 °C, bevorzugt zwischen 190 bis 210 °C und besonders bevorzugt zwischen 200 bis 215 °C beträgt.

Besonders vorteilhaft ist ein erfindungsgemäßes Verfahren, bei dem im Schritt c) der Druck zwischen 1,6 bis 2,5 MPa, bevorzugt zwischen 1,8 bis 2,3 MPa, besonders bevorzugt zwischen 1,9 bis 2,2 MPa beträgt.

Ganz besonders vorteilhaft ist ein erfindungsgemäßes Verfahren, bei dem im Schritt d) die Haltezeit zwischen 0,3 und 3 Stunden, bevorzugt zwischen 0,4 und 2 Stunden und besonders bevorzugt zwischen 0,5 und 1,2 Stunden beträgt.

Das zu separierende Mischprodukt ist gemäß der Wortbedeutung ein künstlich erzeugtes Produkt aus Cellulose und Kunststoff. Das erfindungsgemäße Verfahren ist genau auf die Separation der Bestandteile des Cellulose-Kunststoff-Mischprodukts, nämlich Cellulose und Kunststoff, gerichtet.

Das Besondere des erfindungsgemäßen Verfahrens ist es, dass der abgetrennte Kunststoff dem Reaktionsbehälter in Form von Kugeln entnommen werden kann und dass das verbleibende Cellulosehydrolysat ein vergärbares Substrat darstellt.

Es wurde gefunden, dass die Aufbauagglomeration der Kunststoffe von den Verfahrenparametern einerseits und von der Anlagengeometrie des Rührautoklaven andererseits abhängt. Für die Anlagengeometrie haben die Erfinder ermittelt, dass ein zylindrischer Behälter mit einem Höhe/Durchmesser-Verhältnis von 1,25 : 1,78 bei einer Füllhöhe zwischen 63 % und 81 % zu optimalen Ergebnissen führt. Ebenso ist eine Rührergeometrie optimal, bei welcher ein 2-armiger Blattrührer verwendet wird, dessen Blätter in Wesentlichen eine rechteckige Form und eine Blattanstellung von 15° bis 42° aufweisen, wobei der maximale Durchmesser des Rührers zwischen 60 und 80 % des Innendurchmessers des Rührgefäßes beträgt. Bei dem Blattrührer sind die Rührblätter mittels eines im rechten Winkel zur Rührwelle angeordneten Abstandsstücks von der Rührwelle beabstandet und mittig an der Längsseite der Rührblätter am Abstandshalter befestigt. Der Anstellwinkel ist die Abweichung der Rührblattes von der rechtwinkligen Stellung in Bezug auf die Rührwelle.

Die Erfindung ermöglicht die Aufbauagglomeration des Kunststoffes zu einer oder mehreren Kugeln oder kugelförmigen Gebilden während des Celluloseaufschlusses und deren nachfolgende Separation beispielsweise durch Siebung. Damit entsteht ein stofflich oder energetisch verwertbares Produkt. Zusätzlich erfolgen die Hygienisierung und die Pharmakainhibierung ebenfalls im gleichen Prozess. Der agglomerierte Kunststoff kann nahezu rein und vollständig entnommen werden und eignet sich beispielsweise als hochenergetisches Brennmaterial zur Energieerzeugung. Dieses erhaltene kugelförmige Kunststoffagglomerat stellt somit einen weiteren Gegenstand der Erfindung dar.

Die Cellulose bzw. das Cellulosehydrolysat kann weitergehend verwertet werden. Diese Weiterverarbeitung kann anaerob unter Biogasbildung erfolgen, wobei dem Cellulosehydrolysat stickstoffhaltige Substrate wie beispielsweise Gülle zugesetzt werden können. Gegebenenfalls sind dem Cellulosehydrat auch noch Nährstoffe und/oder Mineralien zuzusetzen. Das Cellulosehydrolysat kann aber auch aerob als Kohlenstoffquelle zur Denitrifikation in Kläranlagen eingesetzt werden. Dieses erfindungsgemäß erhaltene Cellulosehydrolysat stellt somit einen weiteren Gegenstand der Erfindung dar.

Bei allen nach der Behandlung folgenden Schritten werden die im Weiteren benutzten Anlagen nicht durch Pharmaka bzw. Krankheitserreger kontaminiert. Es findet keine zusätzliche Belastung oder Eintritt der Pharmaka bzw. von Krankheitserregern über den Gärrest bzw. den Klärschlamm in die Umwelt statt.

Die Aufgabe der Erfindung wird ferner durch die Bereitstellung einer Anlage gelöst, welche die folgenden Merkmale aufweist.

Die erfindungsgemäße Anlage zur Durchführung eines erfindungsgemäßen Verfahrens und zur stofflichen und/oder energetischen Verwertung der mittels des Verfahrens hergestellten Erzeugnisse besteht aus einem Rührautoklaven, einem Fermenter zur Biogaserzeugung aus dem Cellulosehydrolysat und einem mittels des kugelförmigen Kunststoffagglomerats betreibbaren Kraftwerk, wobei die einzelnen Anlagenteile in an sich bekannter Weise stofflich und/oder energetisch miteinander gekoppelt bzw. verbunden sind.

Erfindungsgemäß Bevorzugt ist es dabei, dass die Anlage weiterhin mindestens einen Anmaischbehälter umfasst.

Die Erfindung wird nun anhand der Figuren und der folgenden Beispiele näher erläutert.

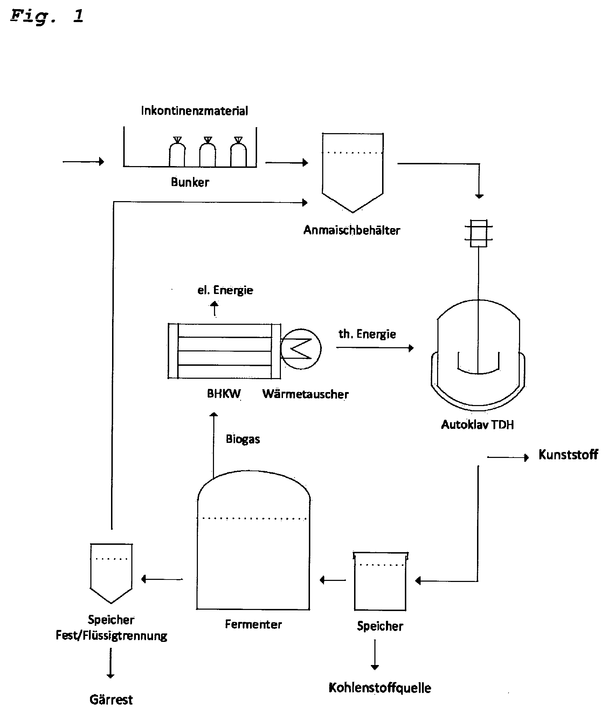

Figur 1 zeigt den Behandlungsprozess sowie die dafür vorgesehene Anlage einschließlich der Thermodruckhydrolyse (TDH) schematisch. Das unsortierte IKM wird angemaischt, sodass ein TR-Gehalt von 5 bis 25 % vorliegt. Die Maische wird dem Autoklaven zur TDH im Batchbetrieb zugeführt. Eine Zerkleinerung ist nicht erforderlich. Lediglich ein Sackaufreißer fördert ein besseres Handling. Die Behandlung erfolgt im Temperaturbereich zwischen 150-250 °C, wobei das Material kontinuierlich gerührt wird. Das Hydrolysat wird nach der Abkühlung dem Fermenter zur Biogasbildung zugeführt. Aus energetischen Gründen bietet sich die thermophile Betriebsweise in einem klassischen einstufigen Fermenter an. Gegebenenfalls kann auch ein Festbettreaktor eingesetzt werden. Die Nutzung des Biogases in einem BHKW führt zur Generierung elektrischer und thermischer Energie. Die Überschusswärme kann dem TDH Prozess zugeführt werden. Der Gärrest ist als Dünger nutzbar bzw. muss weitergehend in Kläranlagen behandelt oder kompostiert werden.

Neben der Erzeugung des Biogases wird der Kunststoff separiert. Die Abtrennung des Kunststoffes ist im TDH-Prozess integriert, da er als Agglomerat zurückbleibt. In Abhängigkeit von Reaktorgestaltung und der Prozessführung ist die Abtrennung nahezu 100%ig.

Aufgrund der eingestellten Temperaturen und Verweilzeiten erfolgt die sichere Hygienisierung und damit Abtötung von Krankheitserregern. Die gesetzlich geforderte Bedingung von v>70 °C für 1 Stunde (Kategorie 3) wird zuverlässig eingehalten.

Der Einsatz des Hydrolysates in einem Fermenter kann als Mono- bzw. Co-Substrat erfolgen. Aufgrund des hohen Kohlenstoffgehaltes und in Abhängigkeit von der Zusammensetzung des Einsatzstoffes, liegt ein C:N-Verhältnis von 40:1 vor. Gegebenenfalls ist daher die Zugabe eines weiteren, stickstoffreichen Substrates bzw. die Zugabe von Spurenelementen erforderlich.

In einem Beispiel erfolgte die Separation des IKM mittels TDH bei einer Aufheizzeit von einer Stunde bei einer Temperatur von 210 °C. Die Haltezeit wurde zwischen 0,3 und 6 Stunden variiert, um eine optimale Expositionsdauer zu ermitteln. Sowohl das beladene IKM, d.h. das Material mit Exkrementen, als auch das unbeladene IKM, wurde getrennt untersucht. Vor der Behandlung wurde das unbeladene IKM mit Wasser, das beladene IKM mit separiertem flüssigen Gärrest des Fermenters angemaischt.

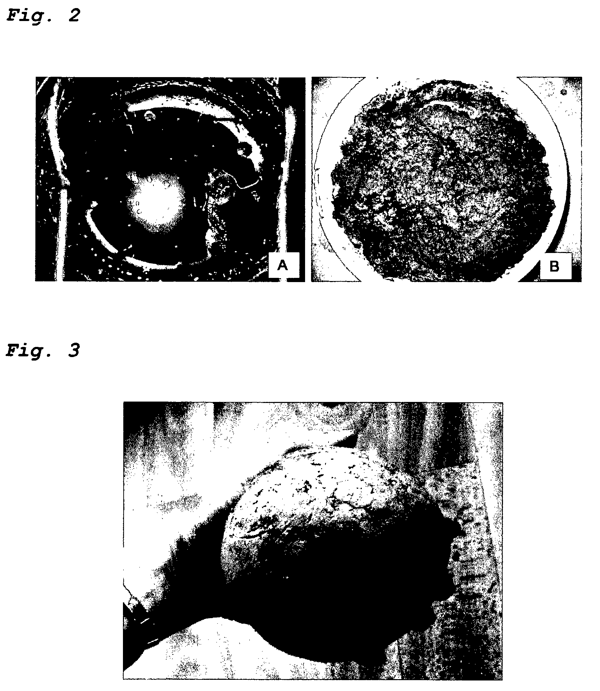

Die Behandlung führte zu einer Änderung der stofflichen Zusammensetzung. In Abhängigkeit von der Haltezeit zersetzte sich die Cellulose, sodass ein pastöses bis flüssiges Hydrolysat entstand. Eine lange Haltezeit von 6 Stunden führte zu einer vollständigen Verflüssigung der Cellulose. Figur 2 zeigt das Hydrolysat nach 1 und nach 6 Stunden Haltezeit. Als zweites Produkt fiel der feste, zu einer Kugel agglomerierte Kunststoff an (Figur 3). Das Kunststoffprodukt konnte leicht manuell, durch Siebung bzw. Schwimm-/Sinktrennung entnommen und stofflich bzw. energetisch verwertet werden. Der Heizwert liegt bei Hᵤ=43,9 MJ/kg und ist damit sehr hoch. Er ist vergleichbar mit dem Heizwert von reinem PP/PE. Im Gegensatz zum Ausgangsmaterial (beladenes IKM, H_{U}=7,5 MJ/kg) ist ebenfalls eine energetische Verwertung gemäß KrW-/AbfG möglich.

**Tabelle 1**

| Vergleich der Heizwerte typischer Brennstoffe und des agglomerierten Kunststoffprodukts. | |
|---|---|
| **Brennstoff** | **Hᵤ [MJ/kg]** |
| Steinkohle | 27-32,7 |
| Braunkohle (Lausitz) | 7,9-9,3 |
| Holzpellets | 18 |
| BRAM | 16-18 |
| Hausmüll | 9-11 |
| reine Cellulose | 17,5 |
| PE | 43 |
| PP | 44 |
| | |
| IKM [7] | 7,5 |
| **Kunststoffprodukt** | **43,9** |

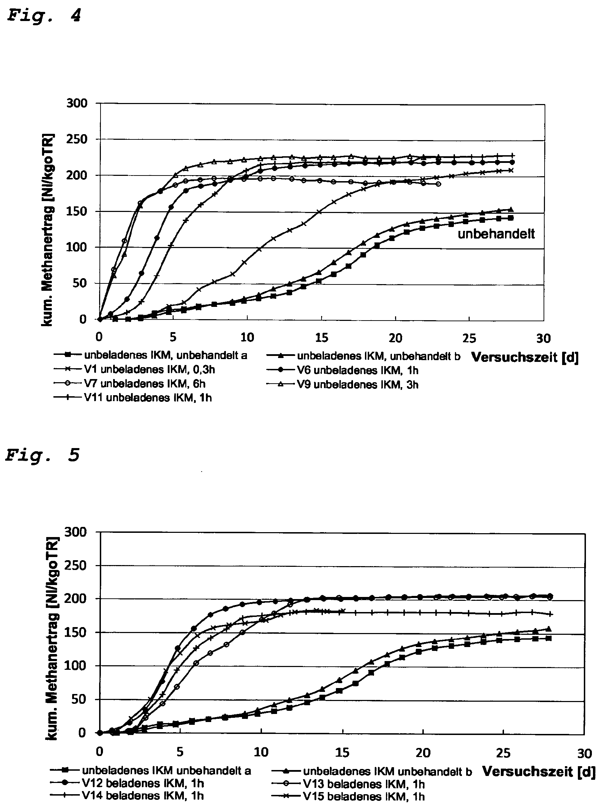

Die Bestimmung der Methanbildung des Hydrolysates erfolgte zum Einen in Batchansätzen durch Standardgärversuche nach VDI 4630. Zum Anderen wurde ein kontinuierlicher Betrieb untersucht. Der zeitliche Verlauf der Gasentwicklung, die Zusammensetzung des Biogases sowie die des Schlammes vor und nach der Vergärung ermöglichten die Beurteilung des Faulverhaltens des Einsatzstoffes hinsichtlich Vergärbarkeit und einer möglichen Hemmung. Figur 4 zeigt den kumulativen Methanertrag des unbeladenen IKM. Die Haltezeit in der TDH hatte offensichtlich einen proportionalen Einfluss auf die Methanbildung.

Der Methanertrag von unbehandeltem IKM lag bei einem geringen Wert von Y=150 NlCH₄/kg oTR. Erst nach einer Verweilzeit von τ=20-25 Tagen sank die Biogasproduktion. Die Referenzkurven zeigen, dass die Methanisierung stark verzögert erfolgte. Durch die Vorbehandlung mittels Thermodruckhydrolyse und einer Haltezeit von 0,3 Stunden konnte der Methanertrag auf Y=210 NlCH₄/kg oTR erhöht werden. Der Abbau erfolgte bereits schneller. Die längere Haltezeit von 1 Stunde brachte eine weitere Erhöhung auf Y=240 NlCH₄/kg oTR. Die erforderliche Verweilzeit war mit τ=5-10 Tagen deutlich geringer, der Abbau war somit deutlich schneller. Die weitere Steigerung der Haltezeit auf 3 Stunden brachte keine Erhöhung des Methanertrages, jedoch eine weitere Verkürzung der Abbauzeit. Die Erhöhung auf 6 Stunden führte sogar zu einer Verringerung des Methanertrages auf Y=190 NlCH₄/kg oTR. Vermutlich kommt es hier bereits zu einer zu starken Zersetzung der organischen Verbindungen, sodass keine weitere biologische Verwertung erfolgen konnte. Eine Haltezeit in der Thermodruckhydrolyse von 1 Stunde ist daher ausreichend und energetisch vorteilhaft. Der erhöhte Energieaufwand einer Behandlung von 3 Stunden bringt lediglich eine Verkürzung der Abbauzeit.

Figur 5 zeigt den Methanertrag des beladenen Inkontinenzmaterials nach der Behandlung. Nach ca. 10 Tagen war bereits ein Methanertrag von Y=200 NlCH₄/kg oTR erreicht. Der Ertrag des unbeladenen IKM lag mit Y=240 NlCH₄/kg oTR (Figur 4) bei gleicher Haltezeit höher. Ursache ist die Zusammensetzung des organischen Trockenrückstandes, da neben der Cellulose ebenfalls Exkremente mit geringerem Biogasertrag vorlagen.

Im kontinuierlichen Betrieb wurde ein gerührter 30-1-Fermenter eingesetzt. Als Substrat erfolgte die Zugabe des Hydrolysates aus beladenem IKM nach einer Behandlung mittels TDH und einer Haltezeit von 1 Stunde. Die Raumbeladung wurde auf b_{R}=1,8 kgoTR/m³ eingestellt und entsprach damit nahezu dem Praxisbetrieb einer klassischen einstufigen Biogasanlage (Nassvergärung). Die Zufuhr erfolgte unter Zugabe von ausgefaulter Rindergülle im Verhältnis 1:1.

Die Zufuhr der Gülle war erforderlich, um die notwendigen Nährstoffe und Spurenelemente vorzulegen. Das C:N-Verhältnis des Hydrolysates war mit 51:1 ungünstig im Vergleich zum optimalen Bereich in der Vergärung von 20-40:1. Da eine starke Abhängigkeit von den Exkrementen und den Fehlwürfen im IKM besteht, wirkt die Zufuhr des Grundsubstrates stabilisierend.

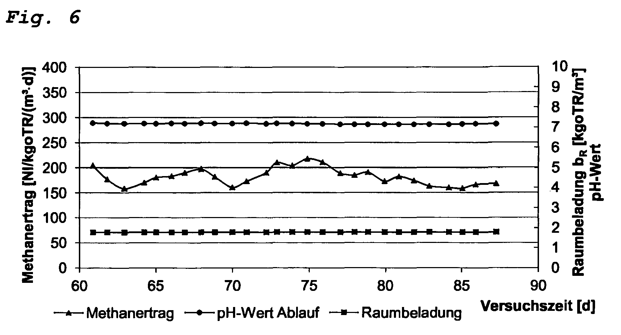

Zuvor erfolgte eine Adaptationsphase von 60 Tagen, sodass eine mikrobiologische Anpassung an das Substrat erfolgen konnte. Figur 6 zeigt den Methanertrag des beladenen IKM im anschließenden Untersuchungszeitraum. Im kontinuierlichen Betrieb lag er im Mittel bei Y=184 NlCH₄/kg oTR. Damit wurde das Ergebnis der Standardgärversuche (Y=200 NlCH₄/kg oTR) nahezu erreicht. Der stabile pH-Wert zeigt, dass im Rahmen dieses Betriebes keine Hemmung eintrat. Ggf. ist eine weitere Erhöhung der Raumbeladung möglich. Der Methangehalt lag bei ca. 50 % und war damit etwas geringer als der theoretische Wert von reiner Cellulose (54 %). Der oTR-Abbau wurde mit 51 % ermittelt. Trotz der Verweilzeit von 31 Tagen lagen daher immer noch Verbindungen vor, die weiterhin schwer oder gar nicht abbaubar sind. Es muss jedoch auch die für gerührte Behälter typische Kurzschlussströmung berücksichtigt werden. Erfindungsgemäß muss die Verweilzeit erhöht werden. Medikamente wurden im Rahmen der Versuche nicht nachgewiesen, jedoch besteht hier eine große Abhängigkeit von der Ausgangszusammensetzung der IKM.

Es konnte gezeigt werden, dass eine sowohl stoffliche als auch energetische Verwertung von beladenem Inkontinenzmaterial möglich ist. Die Vorbehandlung mittels Thermodruckhydrolyse hatte einen positiven Effekt auf die Vergärbarkeit des Inkontinenzmaterials durch die Reduzierung des schwer abbaubaren Celluloseanteils. Dabei erwiesen sich eine Zieltemperatur von 210 °C bei 2,0 MPa und eine Haltezeit von 1 Stunde als vorteilhaft. Parallel erfolgte eine Hygienisierung. Als Produkt lag ein pastöses bis flüssiges Hydrolysat vor, das der Vergärung zugeführt werden konnte.

Der Abbau des behandelten Materials fand mit τ=5-10 Tagen im Vergleich zum unbehandelten Material (τ=25 Tage) deutlich schneller statt. Der Methanertrag im Standardgärversuch konnte um 60 % von Y=150 auf 240 NlCH₄/kg oTR erhöht werden. Damit wurde die Verweilzeit geringer und der Abbaugrad höher.

Der Methanertrag des beladenen IKM im kontinuierlichen praxisnahen Versuch lag nahezu stabil bei Y=184 NlCH₄/kg oTR. Damit wurde der Wert des Standardgärversuches aus diesem Material (Y=200 NlCH₄/kg oTR) nahezu erreicht. Die Methankonzentration lag mit 50 Vol-% im üblichen Bereich für den Einsatz von cellulosehaltigen Verbindungen in einer einstufigen Biogasanlage. Die Verwertung des Biogases kann mittels BHKW erfolgen. Die damit verbundene Überschusswärme kann zum Betrieb der TDH genutzt werden.

Eine unzureichende Vergärung ist durch ein ungünstiges C:N-Verhältnis und gegebenenfalls von zu geringen Nährstoffen und Spurenelementen denkbar. Daher sollte erfindungsgemäß die Co-Fermentation mit weiteren Substraten oder die Zugabe von Spurenelementen erfolgen. Der anfallende flüssige Gärrest kann in einer Kläranlage weitergehend behandelt werden. Weiterhin ist es erfindungsgemäß vorgesehen, das Hydrolysat als Kohlenstoffquelle der Denitrifikation im Rahmen der Abwasserbehandlung zuzuführen.

Ein wesentlicher Vorteil der Thermodruckhydrolyse ist die nahezu vollständige und integrierte Abtrennung des Kunststoffes aus dem Inkontinenzmaterial. Er ist nahezu fremdstofffrei und kann als Agglomerat abgetrennt und weitergehend verwertet werden. Es ist sowohl die stoffliche als auch energetische Verwertung möglich. Der Heizwert des Kunststoffes liegt bei Hᵤ=43,9 MJ/kg. Er ist wesentlich höher als der Heizwert des Ausgangsstoffes, sodass eine thermische Verwertung im Sinne des KrW-/AbfG möglich ist.

### Beispiel

Das angemaischte Inkontinenzmaterial wird einem Autoklaven zur Thermodruckhydrolyse im Batch-Betrieb zugeführt. Eine Zerkleinerung ist nicht erforderlich. Die Behandlung erfolgt im Temperaturbereich zwischen 180-220 °C. Der Druckbereich liegt zwischen 1,6-2,5 MPa. Nach einer Haltezeit von 0,3-6 Stunden wird das Material abgekühlt. Während des gesamten Prozesses erfolgt eine Durchmischung des Materials mittels eines zentralen Rührwerkes mit Propellerblatt bei eine definierten Drehzahl von 50 bis 350 min⁻¹. Das Verhältnis der angegebenen Verfahrensparameter ist für die Aufbauagglomeration entscheidend.

Nach dem Prozess wird der zu einer oder mehreren Kugeln agglomerierte Kunststoff während des Ablassvorganges durch ein Sieb entnommen. Das pastöse bis flüssige Cellulosehydrolysat ist hygienisiert, frei von Arzneimittelrückständen und kann weitergehend aerob oder anaerob verwertet werden.

### Literatur:

[1] Georg Consult: Pflegeheim-Atlas & Bedarfsprognose Deutschland 2009-2025
[2] Destatis: Bevölkerung Deutschlands bis 2060, Statistisches Bundesamt, Wiesbaden, 2009
[3] Studie medicus Cottbus GmbH, Cottbus, 2011
[4] Schalles, H.: Die stoffliche und energetische Verwertung von Kunststoffabfällen, Vortrag an den 9. Schweriner Wissenschaftstagen, 10.09.2004
[5] Bischofsberger, W.: Anaerobtechnik, Springer, 2005
[6] Barlindhaug, J., Odegaard, H.: Thermal hydrolysis for production of carbon sources for denitrificaion, wat sci tec, Vol. 34, No. 1-2, pp. 371-378, 1996
[7] Meyer, P., Meyer, Ö.: Ökologische Bilanz der Entsorgung von Inkontinenz-System-Abfall aus öffentlichen Einrichtungen, Müll und Abfall, Heft 5, 2001

## Patentansprüche

1. Verfahren zur Separation von Cellulose-Kunststoff-Mischprodukten, wobei man die folgenden Schritte durchführt:
a) Anmaischen der Cellulose-Kunststoff-Mischprodukte mit Wasser;
b) Einbringen der im Schritt a) erzeugten Maische in einen Rührautoklaven und Verschließen des Rührautoklaven;
c) Erwärmen der Maische unter fortgesetztem Rühren mit einer Drehzahl von 50 bis 350 min⁻¹, auf eine Temperatur von 150 bis 250 °C, bei einem Druck von 1,2 bis 3,0 MPa;
d) Halten der Temperatur und des Drucks der im Schritt c) erwärmten Maische unter fortgesetztem Rühren für eine Haltezeit von 0,3 bis 6 Stunden, wobei die Cellulose hydrolysiert und die Kunststoffe zu kugelförmigen Gebilden agglomerieren;
e) Abkühlen, Entspannen und Öffnen des Rührautoklaven.

2. Verfahren gemäß Anspruch 1, **dadurch gekennzeichnet, dass** im Schritt c) die Rührerdrehzahl zwischen 100 bis 300 min⁻¹, bevorzugt zwischen 120 bis 290 min⁻¹ und besonders bevorzugt zwischen 140 bis 260 min⁻¹ liegt.

3. Verfahren gemäß Anspruch 1, **dadurch gekennzeichnet, dass** im Schritt c) die Temperatur zwischen 180 bis 220 °C, bevorzugt zwischen 190 bis 210 °C und besonders bevorzugt zwischen 200 bis 215 °C beträgt.

4. Verfahren gemäß Anspruch 1, **dadurch gekennzeichnet, dass** im Schritt c) der Druck zwischen 1,6 bis 2,5 MPa, bevorzugt zwischen 1,8 bis 2,3 MPa, besonders bevorzugt zwischen 1,9 bis 2,2 MPa beträgt.

5. Verfahren gemäß Anspruch 1, **dadurch gekennzeichnet, dass** im Schritt d) die Haltezeit zwischen 0,3 und 3 Stunden, bevorzugt zwischen 0,4 und 2 Stunden und besonders bevorzugt zwischen 0,5 und 1,2 Stunden beträgt.

6. Cellulosehydrolysat, hergestellt nach einem Verfahren gemäß Anspruch 1.

7. Kugelförmiges Kunststoffagglomerat, hergestellt nach einem Verfahren gemäß Anspruch 1.

8. Anlage zur Durchführung eines Verfahrens gemäß Anspruch 1 und zur stofflichen und/oder energetischen Verwertung der mittels des Verfahrens hergestellten Erzeugnisse gemäß den Ansprüchen 6 und 7, bestehend aus einem Rührautoklaven, einem Fermenter zur Biogaserzeugung aus dem Cellulosehydrolysat und einem mittels des kugelförmigen Kunststoffagglomerats betreibbaren Kraftwerk, wobei die einzelnen Anlagenteile in an sich bekannter Weise stofflich und/oder energetisch miteinander gekoppelt bzw. verbunden sind.

9. Anlage, gemäß Anspruch 8, **dadurch gekennzeichnet, dass** weiterhin ein Anmaischbehälter vorgesehen ist.

10. Verwendung eines Cellulosehydrolysats gemäß Anspruch 6, zur Erzeugung von Biogas mittels Nassvergärung.

11. Verwendung gemäß Anspruch 10, wobei die Erzeugung des Biogases unter Zusatz von Gülle und/oder anderen stickstoffhaltigen Substraten erfolgt.

12. Verwendung eines Cellulosehydrolysats gemäß Anspruch 6, zur Denitrifikation in Kläranlagen.

13. Verwendung eines kugelförmiges Kunststoffagglomerats gemäß Anspruch 7, zur Erzeugung von Energie durch Verbrennung.

## Claims

1. Method for separating mixed cellulose-plastic products wherein the following steps are performed:
a) Mashing of the mixed cellulose-plastic products with water;
b) Introducing the mash produced in step a) into a stirred autoclave and closing the stirred autoclave;
c) Heating the mash during continuous stirring at a speed of 50 to 350 rpm to a temperature of 150 to 250°C at a pressure of 1.2 to 3.0 MPa;
d) Holding the temperature and the pressure of the mash heated in step c) during continuous stirring for a hold time of 0.3 to 6 hours, wherein the cellulose is hydrolysed and the plastics are agglomerated to spherical objects;
e) Cooling down, pressure relieving and opening of the stirred autoclave.

2. Method, according to claim 1, **characterised in that** the stirrer speed in step c) lies between 100 and 300 rpm, preferred between 120 and 290 rpm and especially preferred between 140 and 260 rpm.

3. Method, according to claim 1, **characterised in that** the temperature in step c) lies between 180 to 220°C, preferred between 190 to 210°C and especially preferred between 200 to 215°C.

4. Method, according to claim 1, **characterised in that** the pressure in step c) lies between 1.6 to 2.5 MPa, preferred between 1.8 to 2.3 MPa and especially preferred between 1.9 to 2.2 Mpa.

5. Method, according to claim 1, **characterised in that** the hold time in step d) lies between 0.3 and 3 hours, preferred between 0.4 and 2 hours and especially preferred between 0.5 and 1.2 hours.

6. Cellulose hydrolysate, produced by a method according to claim 1.

7. Spherically-shaped plastic agglomerate, produced by a method according to claim 1.

8. Plant for performing a method according to claim 1 and for material and/or energy recovery of prepared products according to claims 6 and 7 by means of the said method, comprising a stirred autoclave, a fermenter for producing biogas from the cellulose hydrolysate and a power plant that can be operated using the spherical plastic agglomerate, wherein the individual plant sections are coupled or connected materially and/or energetically to each other in a known manner.

9. Plant, according to claim 8, **characterised in that** a mashing tank is also provided.

10. Use of cellulose hydrolysate according to claim 6, for the production of biogas by means of wet fermentation.

11. Use, according to claim 10, wherein the production of biogas takes place with the addition of semi-liquid manure and/or other nitrogenous substrates.

12. Use of a cellulose hydrolysate according to claim 6 for denitrification in sewage treatment plants.

13. Use of a spherically-shaped plastic agglomerate according to claim 7 for the production of energy by incineration.

## Revendications

1. Procédé de séparation de produits mélangés de cellulose-matière plastique, où sont exécutées les étapes suivantes:
a) trempage des produits mélangés de cellulose-matière plastique avec l'eau;
b) dépose de la trempe obtenue en étape a) dans un autoclave à brassage et fermeture de l'autoclave à brassage;
c) chauffage de la trempe en poursuivant l'agitation avec une vitesse comprise entre 50 et 350 min⁻¹, à une température de 150 à 250° C, sous pression de 1,2 à 3,0 MPa;
d) maintien de la température et de la pression de la trempe chauffée pendant l'étape c) en poursuivant agitation pendant un temps de maintien compris entre 0,3 et 6 heures, la cellulose étant hydrolysée et les matières plastiques s'agglomérant sous forme de structures sphériques;
e) refroidissement, détente et ouverture de l'autoclave à brassage.

2. Procédé selon la revendication 1, **caractérisé en ce que** la vitesse d'agitateur en étape c) est comprise entre 100 et 300 min⁻¹, avantageusement entre 120 et 290 min⁻¹ et préférentiellement entre 140 et 260 min⁻¹.

3. Procédé selon la revendication 1, **caractérisé en ce que** la température en étape c) est comprise entre 180 et 220°C, avantageusement entre 190 et 210°C et préférentiellement entre 200 et 215°C.

4. Procédé selon la revendication 1, **caractérisé en ce que** la pression en étape c) est comprise entre 1,6 et 2,5 MPa, avantageusement entre 1,8 et 2,3 MPa, préférentiellement entre 1,9 et 2,2 MPa.

5. Procédé selon la revendication 1, **caractérisé en ce que** le temps de maintien en étape d) est compris entre 0,3 et 3 heures, avantageusement entre 0,4 et 2 heures et préférentiellement entre 0,5 et 1,2 heures.

6. Hydrolysat de cellulose, fabriqué conformément à un procédé selon la revendication 1.

7. Agglomérat sphérique de matière plastique, fabriqué conformément à un procédé selon la revendication 1.

8. Installation pour l'exécution d'un procédé selon la revendication 1 et pour l'utilisation matériel et/ou énergétique des produits fabriqués selon les revendications 6 et 7 au moyen du procédé, composée d'un autoclave à brassage, d'un digesteur pour la génération de biogaz à partir de l'hydrolysat de cellulose et d'une centrale électrique pouvant fonctionner avec l'agglomérat sphérique de matière plastique, les différentes parties d'installation étant accouplées ou raccordées entre elles de manière connue pour l'utilisation matériel et/ou énergétique.

9. Installation, selon la revendication 8, **caractérisée en ce qu'**un conteneur de trempage est en outre prévu.

10. Utilisation d'un hydrolysat de cellulose selon la revendication 6 pour la génération de biogaz par fermentation humide.

11. Utilisation selon la revendication 10, où le biogaz est généré par ajout de lisier et/ou d'autres substrats azotés.

12. Utilisation d'un hydrolysat de cellulose selon la revendication 6 pour la dénitrification dans des stations d'épuration.

13. Utilisation d'un agglomérat sphérique de matière plastique selon la revendication 7 pour la génération d'énergie par combustion.
